(19) Europäisches Patentamt
European Patent Office
Office européen des brevets

(11) EP 4 327 810 A1

(12) **EUROPEAN PATENT APPLICATION**
published in accordance with Art. 153(4) EPC

(43) Date of publication:
**28.02.2024 Bulletin 2024/09**

(21) Application number: **22791551.9**

(22) Date of filing: **30.03.2022**

(51) International Patent Classification (IPC):
***A61K 31/4745*** (2006.01) ***A61P 3/04*** (2006.01)
***A61P 3/06*** (2006.01) ***A61P 43/00*** (2006.01)

(52) Cooperative Patent Classification (CPC):
**A61K 31/4745; A61P 3/04; A61P 3/06; A61P 43/00**

(86) International application number:
**PCT/JP2022/016109**

(87) International publication number:
**WO 2022/224776 (27.10.2022 Gazette 2022/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.04.2021 JP 2021072843**

(71) Applicant: **MITSUBISHI GAS CHEMICAL COMPANY, INC.**
**Chiyoda-ku**
**Tokyo 100-8324 (JP)**

(72) Inventors:
- **TSUJI, Syafiqah**
**Niigata-shi, Niigata 950-3112 (JP)**
- **IKEMOTO, Kazuto**
**Niigata-shi, Niigata 950-3112 (JP)**

(74) Representative: **Müller-Boré & Partner Patentanwälte PartG mbB**
**Friedenheimer Brücke 21**
**80639 München (DE)**

(54) **LIPID DECREASE PROMOTER**

(57) The present invention provides an agent for promoting lipid reduction, containing IPQ and/or a salt thereof as an active ingredient.

Figure 1:

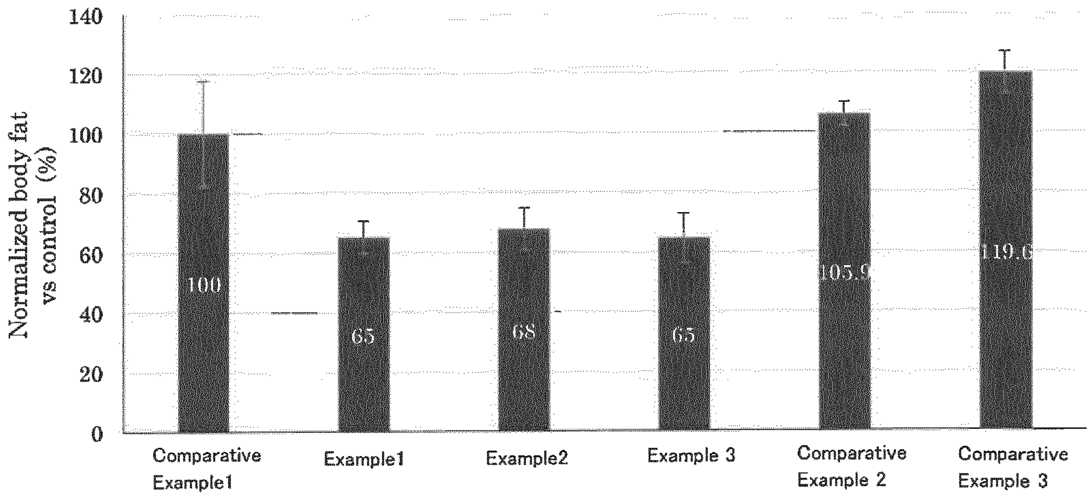


EP 4 327 810 A1

## Description

### Technical Field

The present invention relates to a novel agent for promoting lipid reduction and a method for producing the same, a composition containing the agent for promoting lipid reduction, and a method for promoting lipid reduction.

### Background Art

Obesity is one of the most serious diseases in modern society, and the principal cause thereof is excessive ingestion of fat. It is known that excessive ingestion of fat causes not only obesity but also lifestyle diseases caused by obesity such as metabolic syndrome, diabetes, hyperlipidemia, hypertension, and arteriosclerosis.

As a therapeutic drug for obesity, an appetite suppressant such as mazindol, and a lipase inhibitor such as orlistat are used. These medicaments have been, however, reported to cause side effects such as dry mouth, constipation, nausea, insomnia, headache, and palpitation as well as oil spot, flatulence, sudden bowel movement, increase of bowel movement, fecal incontinence, rectal leakage, upper respiratory infection, and steatorrhea, and thus, cannot be always sufficiently safe.

In order to prevent obesity, it is effective to reduce calorie intake by dietary restrictions. For this purpose, however, sufficient nutrition education should be provided, and hence, it is substantially difficult to employ this method in everyday life in many cases. Therefore, a substance that is sufficiently safe, and can efficiently decrease obesity is being strongly required.

Imidazopyrroloquinoline (hereinafter referred to also as "IPQ") can be in a structure having various substituents as a reaction product of coenzyme pyrroloquinoline quinone and an amino acid (Non Patent Literature 1). The properties thereof are different from those of pyrroloquinoline quinone, and it is neither oxidized nor reduced, and has largely different properties. Therefore, it has been reported that it does not have activity as that of pyrroloquinoline quinone (Non Patent Literature 2).

On the other hand, it has been revealed, through tests using cells, that IPQ has an antioxidative effect (Non Patent Literature 3).

### Citation List

### Non Patent Literature

Non Patent Literature 1: BioFactors, Vol.5, No. 2, pp75-81, 1995/1996
Non Patent Literature 2: J Cell Sci. 2017 Aug 1; 130 (15): 2631-2643. doi: 10.1242/jcs.202119
Non Patent Literature 3: Heliyon, volume 6, e03240, 2020
Non Patent Literature 4: Environ Health Perspect, 2015; 123 (8): 813-819

### Summary of Invention

### Technical Problem

It is, however, not known to use this IPQ for decreasing obesity, and it has not been reported to use it as an agent for promoting lipid reduction.

An object of the present invention to provide, by using the above-described IPQ as a substance useful for decreasing obesity, a novel agent for promoting lipid reduction that is safe and capable of efficiently promoting lipid reduction, a production method thereof, a composition containing the agent for promoting lipid reduction, and a method for promoting lipid reduction.

### Solution to Problem

In order to solve the above-described problems, the present inventors have made earnest studies, resulting in surprisingly finding that IPQ and/or a salt thereof has a function to promote lipid reduction. Based on this finding, the present inventors have made further studies, and thus, the present invention has been accomplished.

Specifically, the present invention provides the following:

[1] An agent for promoting lipid reduction, comprising imidazopyrroloquinoline represented by Formula 1 and/or a salt thereof as an active ingredient:

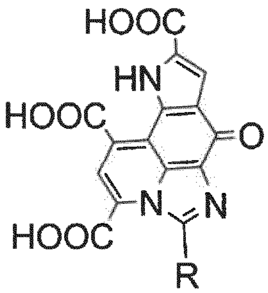

・・・Formula 1

wherein R represents an amino acid residue.

[2] The agent according to [1], wherein R is a glycine residue, an alanine residue, an arginine residue, a histidine residue, an isoleucine residue, a leucine residue, a lysine residue, a serine residue, a threonine residue, a tryptophan residue, a tyrosine residue, or a valine residue.

[3] The agent according to [2], wherein R is a glycine residue, a leucine residue, or an arginine residue.

[4] The agent according to any one of [1] to [3], to be ingested during exercise with an exercise intensity of 3 METs or more, or within 1 hour before starting or within 1 hour after ending the exercise.

[5] The agent according to any one of [1] to [4], wherein the promoting lipid reduction is body fat metabolism promotion.

[6] A composition, comprising the agent according to any one of [1] to [5].

[7] A method for promoting lipid reduction, using the agent according to any one of [1] to [5].

[8] A growth promoter, comprising imidazopyrroloquinoline represented by Formula 1, and/or a salt thereof as an active ingredient:

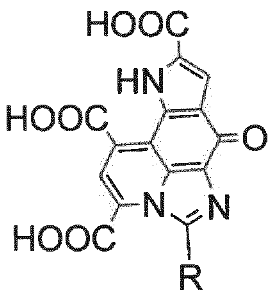

・・・Formula 1

wherein R represents an amino acid residue.

## Advantageous Effects of Invention

According to the present invention, a novel agent for promoting lipid reduction that is safe and capable of efficiently promoting lipid reduction, a production method thereof, a composition containing the agent for promoting lipid reduction, and a method for promoting lipid reduction can be provided.

Besides, according to the present invention, a novel growth promoter can be provided.

In addition, since IPQ and/or a salt thereof used in the present invention is not cytotoxic, it can be regarded that an agent and a composition of the present invention are sufficiently safe for animals including humans.

## Brief Description of Drawings

[Figure 1] Figure 1 is a graph illustrating comparison in body fat mass in daphnids between examples and comparative examples.

[Figure 2] Figure 2 illustrates the relationship between the logarithm of feed and fat mass of daphnids.

[Figure 3] Figure 3 illustrates LC-MS analysis results obtained using H-IPQ of Production Example 1.

[Figure 4] Figure 4 illustrates LC-MS analysis results obtained using Arg-IPQ of Production Example 2.

[Figure 5] Figure 5 illustrates LC-MS analysis results obtained Using Leu-IPQ of Production Example 3.

Description of Embodiments

Embodiments for practicing the present invention will now be described in detail, and it is noted that the present invention is not limited to these embodiments but various modifications and changes can be made without departing from the scope thereof.

[Embodiment 1: Agent for Promoting Lipid Reduction]

During the process of studying the physiological functions of IPQ, the present inventors grew small crustaceans under conditions with IPQ added, and found that IPQ has a function of promoting lipid reduction. In other words, an agent for promoting lipid reduction is based on this finding, and contains IPQ and/or a salt thereof as an active ingredient.
IPQ used in the present embodiment is a compound having a chemical structure shown in the following Formula 1:

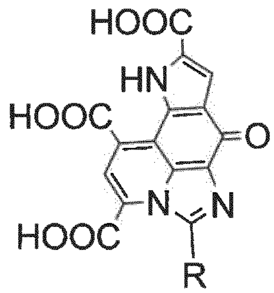

· · · Formula 1

wherein R represents an amino acid residue.
In Formula 1, R represents a residue resulting from a reaction of pyrroloquinoline quinone with an amino acid (referred to as the "amino acid residue"). The amino acid residue represented by R is not especially limited, and examples include a glycine residue, an alanine residue, an arginine residue, an asparagine residue, an aspartic acid residue, a cysteine residue, a glutamine residue, a glutamic acid residue, a histidine residue, an isoleucine residue, a leucine residue, a lysine residue, a methionine residue, a phenylalanine residue, a serine residue, a threonine residue, a tryptophan residue, a tyrosine residue, and a valine residue.
Among these, R is preferably a glycine residue, an alanine residue, an arginine residue, a histidine residue, an isoleucine residue, a leucine residue, a lysine residue, a serine residue, a threonine residue, a tryptophan residue, a tyrosine residue, or a valine residue, and more preferably a glycine residue, a leucine residue, or an arginine residue. When R is a glycine residue, R represents a hydrogen atom. When such an amino acid residue is used, the effect of promoting the lipid reduction tends to be further improved.
A salt of IPQ is a salt formed by a carboxyl group in Formula 1 with an alkali metal ion or the like. The salt of IPQ is not especially limited, and examples include alkali metal salts such as sodium and potassium (specifically, IPQ sodium, IPQ potassium and the like), alkaline earth metal salts such as calcium and magnesium (specifically, IPQ calcium, IPQ magnesium and the like), IPQ aluminum, IPQ zinc, IPQ manganese, and IPQ iron. Among these, the salt of IPQ is preferably IPQ sodium or IPQ calcium. Particularly, as a sodium salt, monosodium, disodium, or trisodium can be used, and among these, IPQ disodium is easily available and easily used. In the present embodiment, the salt of IPQ may be a hydrate. Alternatively, it may contain a crystal solvent.
IPQ and a salt thereof used in the present invention can be obtained through a reaction between an amino acid and pyrroloquinoline quinone. IPQ thus obtained has an amino acid residue, that is, a substituent inherent to the amino acid used. A method for obtaining IPQ or a salt thereof is not especially limited, and either of a natural one derived from an animal or a plant, and one obtained by a chemical synthesis method, a fermentation method or the like may be used. Based on the impurity, the production cost and the like of IPQ or a salt thereof to be obtained, a favorable method for producing the IPQ or the salt thereof can be appropriately selected.
Herein, the term "agent for promoting lipid reduction" means an agent having a function to promote lipid reduction in a living body.
Herein, the term "lipid" is a general term for water-insoluble substances present in a living body. Besides, lipid to be metabolized may be a solid or a liquid at normal temperature, and is preferably a solid. It is noted that normal temperature usually refers to 15 to 25°C in accordance with the general rules in The Japanese Pharmacopoeia 16th edition. Examples of the lipid include simple lipids (such as neutral lipid), complex lipids (such as phospholipid and

glycolipid), and derived lipids (such as fatty acid and cholesterol). Among these, the lipid used as a target in Embodiment 1 is preferably simple lipids, and among these, neutral lipid is more preferred, and triacylglycerol (triglyceride) is most preferred.

Herein, the term "promotion of reduction" of lipid refers mainly to decomposition of the lipid, but is not limited thereto as long as the amount of the lipid can be reduced.

Herein, the term "promotion of lipid reduction" includes the concept of reducing lipid within a living body by decomposing the lipid (also referred to as "promotion of lipid metabolism" or "fat burning" in some cases), and the concept of suppressing accumulation of lipid within a living body by, for example, reducing lipid ingested from outside the body.

Among these, the promotion of lipid reduction in Embodiment 1 is preferably promotion of lipid metabolism for reducing lipid within a living body by decomposing the lipid. Examples of the lipid include, in accordance with the position in a living body, body fat (such as subcutaneous fat, or visceral fat), and blood lipid, and the promotion is preferably promotion of body fat metabolism for reducing body fat within a living body preferably by decomposing the body fat. From the viewpoint of the type of a compound of lipid, the lipid reduction in Embodiment 1 is preferably promotion of neutral lipid metabolism.

The agent of Embodiment 1 is useful, owing to the lipid reduction promoting function, as food or a medicament for preventing and treating obesity (also designated as "adiposity"), promoting liver fat reduction, promoting body fat reduction and the like. In addition, the agent of Embodiment 1 is useful also as food or a medicament for preventing and treating a disease caused by obesity. Such a disease is not especially limited, and examples include metabolic syndrome, fatty liver, diabetes (including type 2 diabetes mellitus), hyperlipidemia, hypertension, and arteriosclerosis.

The agent of Embodiment 1 is used preferably together with exercise. More specifically, the agent is preferably ingested during exercise with an exercise intensity of 3 METs or more, or within 1 hour before starting or within 1 hour after ending the exercise. When the agent of Embodiment 1 is administered or ingested at this timing, lipid metabolism caused by exercise is more efficiently promoted.

"METs" is a unit indicating an exercise intensity, and is obtained as an index indicating how many times the energy consumption in one physical activity is larger than the energy consumption during rest. As for METs, an oxygen amount necessary for retaining a resting state (oxygen demand) is set with 3.5 ml/kg/min used as one unit regardless of sex and weight. Although not limited, for example, a resting state of sitting corresponds to 1 MET, ordinary walking corresponds to 3 METs, and an intensity of 3 METs is regarded as an intermediate intensity. In Embodiment 1, the type of exercise employed together with the agent is not especially limited as long as it is physical activity of an exercise intensity of 3 METs or more.

[Embodiment 2: Growth Promoter]

During the process of studying the physiological functions of IPQ, the present inventors grew small crustaceans under conditions with IPQ added, and found that IPQ has a growth promoting function. In other words, a growth promoter is based on this finding, and contains IPQ and/or a salt thereof as an active ingredient.

It is noted that IPQ and/or a salt thereof used in the growth promoter of Embodiment 2 are the same as those described above.

Herein, the term "growth promoter" means an agent having a function of promoting growth of a living body. Herein, the term "growth" refers to increase of a body size, and a rate of the increase.

[Use Form]

A use method of the agent of the present embodiment is not especially limited as long as it is a method employed for promotion of lipid reduction or improvement of growth promotion, and the agent can be used as, for example, food, functional food, a medicament, or a quasi-drug for humans or animals. The functional food herein means food ingested for purpose of health maintenance or as nutritional support instead of a meal, such as health food, a nutritional supplement, food with nutrient functional claims, and food for health uses. Examples of a specific form include, but are not limited to, a capsule, a tablet candy, a chewable tablet, a tablet, and a drink.

In use as a medicament, the agent of the present embodiment can be used by a method of oral administration, injection, intravenous drip, or percutaneous absorption. For oral administration, the agent of the present embodiment can be used in the form of a hard capsule, a soft capsule, or a tablet of a mixture with another substance. Alternatively, the agent of the present invention can be used as a beverage, an infusion, or an injection.

As a preferable form among these, the agent of the present embodiment is orally ingested in the form of a beverage, a soft capsule, or a tablet.

The agent of the present embodiment is useful as a medicament and food, and can be applied to a mammal. Examples of the mammal include primates (such as a human, a monkey, and a chimpanzee), rodents (such as a mouse, a rat, and a guinea pig), pets (such as a dog, a cat, and a rabbit), and working animals and domestic animals (such as

a cow, a horse, a pig, sheep, and a goat), and a human is preferred in the present embodiment. When the agent is applied to a mammal excluding a human, a dose (intake) of the agent of the present embodiment may be appropriately adjusted in accordance with the weight or the size of the animal.

[Composition]

The agent of the present embodiment can be formulated, if pharmaceutically necessary, by an ordinary method using a pharmaceutically acceptable carrier into a composition (hereinafter referred to as the "composition of the present embodiment"). Thus, it can be said that the composition of the present embodiment contains the agent of the present embodiment. The composition of the present embodiment contains the agent of the present embodiment, and hence can possess all the effects exhibited by the agent of the present embodiment. Specifically, the composition of the present embodiment possesses the lipid reduction promoting function and the growth promoting function. Here, the terms "lipid reduction promotion" and "growth promotion" mean the same as those described above.

The carrier is not especially limited but can be appropriately selected in accordance with a dosage form of the preparation (composition), and examples include additives such as an excipient, a binder, a lubricant, a disintegrating agent, a solvent, a stabilizer, a dissolution auxiliary agent, an antioxidant, a colorant, a flavoring agent, and a sweetener.

The excipient is not especially limited, and examples include sugars (such as sucrose, lactose, glucose, and mannitol), starch (such as corn starch), crystalline cellulose, potassium phosphate, potassium sulfate, and magnesium sulfate.

The binder is not especially limited, and examples include pregelatinized starch, gelatin, gum tragacanth, gum arabic, methylcellulose, carboxymethyl cellulose, sodium carboxymethyl cellulose, crystalline cellulose, hydroxypropyl cellulose, hydroxypropyl methylcellulose, polyvinyl pyrrolidone, and polyvinyl alcohol.

The lubricant is not especially limited, and examples include magnesium stearate, talc, polyethylene glycol, silica, and hydrogenated vegetable oils.

The disintegrating agent is not especially limited, and examples include starch, crystalline cellulose, carboxymethyl cellulose, agar, calcium citrate, calcium carbonate, sodium bicarbonate, and dextrin.

The solvent is not especially limited, and examples include water, ethanol, glycerol, saline, and a soybean oil.

The stabilizer is not especially limited, and examples include benzoic acid, sodium benzoate, ethyl parahydroxybenzoate, and propylene glycol.

The dissolution auxiliary agent is not especially limited, and examples include fumaric acid, succinic acid, and malic acid.

The antioxidant is not especially limited, and examples include ascorbic acid, tocopherol, sodium hydrogen sulfite, sodium thiosulfate, sodium pyrosulfite, and citric acid.

Examples of the colorant, the flavoring agent, and the sweetener include those usually allowed to be added in the fields of medicaments and food.

The content of IPQ and/or a salt thereof in the composition of the present embodiment is preferably 0.10 to 99.99% by mass, more preferably 0.60 to 99.90% by mass, and further preferably 1.00 to 99.90% by mass based on the total weight of the composition of the present embodiment. The content of IPQ and/or a salt thereof in the agent of the present embodiment is dealt with as the weight of the substance. Accordingly, there is no need to convert the salt of IPQ to an educt (free body) of IPQ, and when the salt of IPQ is in the form of a hydrate, the weight is calculated as the form including a water molecule.

The composition of the present embodiment can be used as a medicament (namely, a pharmaceutical composition). In this case, the pharmaceutical composition of the present embodiment can be dealt with as a preventive or therapeutic agent for diseases related to adiposity, and examples of these diseases are the same as those described above. The composition of the present embodiment can be dealt with also as a therapeutic agent related to growth promotion.

An administration method of the pharmaceutical composition of the present embodiment is not especially limited, and an oral administration method is generally employed, and in accordance with the state, severity and the like of a target patient, parenteral administration (such as intravascular (intravenous or intraarterial) administration, subcutaneous administration, transdermal administration, or intrarectal administration) may be appropriately selected. Since IPQ and/or a salt thereof corresponding to the active ingredient are all edible, oral administration is preferably employed in the present embodiment.

The dosage form of the pharmaceutical composition of the present embodiment is not especially limited, and a form suitable to the administration method can be employed. Examples of the form suitable to oral administration include a tablet, a lozenge, a hard or soft capsule, an aqueous or oily suspension, an emulsion, a dispersible powder or granule, a syrup, and an elixir. As for the form suitable to parenteral administration, examples of the form suitable to, for example, parenteral injection include a sterile solution, a suspension, an emulsion and the like for intravenous, subcutaneous, intramuscular, intravascular, or injection administration, and examples of the form suitable to transdermal

administration include a cream, an ointment, a gel, and an aqueous or oily liquid (including a suspension).

The composition of the present embodiment can be used also in the form of food or drink (namely, a food or drink composition). In this case, the food or drink composition of the present embodiment can be formulated by an ordinary method using not only the above-described pharmaceutically acceptable carrier but also a carrier usually used in the field of food or drink.

The food or drink of the present embodiment means the whole food and drink, and encompasses, in addition to general food including what is called heath food, food for special dietary uses (such as food for specified health uses, food for the sick, and food for people with dysphagia) prescribed by Consumer Affairs Agency, and food with nutrient function claims, and in addition, supplements, feed and the like are also encompassed in the food or drink of the present embodiment.

The food or drink composition of the present embodiment is not especially limited in the dosage form, and can be in any of dosage forms of a fine granule, a granule, a pill, tablets (including a coated tablet and a sugar coated tablet), capsules (including a hard capsule, a soft capsule, and a microcapsule), a beverage, a drink, liquids (including a syrup, an emulsion, and a suspension), powdered food, a jelly, and a candy.

A dose (intake) of the agent and the composition of the present embodiment is not especially limited, and may be in a range of an effective dose capable of exhibiting the lipid reduction promoting function or the growth promoting function in a body, and can be appropriately set in accordance with an individual difference of the target, the symptom, the administration method and the like. A daily dose (intake) can be administered (ingested) at one time, or dividedly several times (for example, twice or three times). The agent and the composition of the present embodiment may be administered (ingested) at any timing of before meal, after meal, and between meals, and the period is not especially limited as long as the effects of the present embodiment are exhibited. Hereinafter, the term "administration" used herein has the meaning of encompassing the concept of the term "ingestion".

As described above, IPQ and/or a salt thereof can be formulated into separate preparations, and in this case, these preparations can be used together in the present embodiment.

When the agent or the composition of the present embodiment is used, an existing component having the lipid reduction promoting function or the growth promoting function can be used together. In such a case, as for the order of administering the agent or the composition of the present embodiment and the exhibiting component, these may be administered at the same timing or at different timings. When the different timings are employed, the agent or the composition of the present embodiment may be administered either before or after the existing component.

The existing component related to the lipid reduction promoting function is not especially limited, and examples include, but are not limited to, a tea extract, a cacao extract, a coffee-derived mannooligosaccharide, an apple-derived procyanidin, glucosyl hesperidin, resveratrol, a green citrus extract, citrus naringin, glucosamine, mushroom chitosan, chitosan, diacylglycerol, medium chain fatty acid, EPA, DHA, soy protein, plant sterol (β-sitosterol), psyllium seed coat-derived dietary fiber, low molecular weight sodium alginate, guarana, ginger, garcinia, globin digest, carnitine, α-lipoic acid, plant stanol ester, phospholipid-bound soy peptide, and a combination of these.

## Examples

The present invention will now be more specifically described by way of examples, and it is noted that these examples are merely illustrative and do not limit the scope of the present invention at all.

IPQ used in the present examples had the following structure, and was prepared in accordance with any of Production Examples 1 to 3 described below. LC-MS analysis was employed for identifying a substance, and the measurement conditions were set as follows: Apparatus: Alliance 22695 (Waters); eluent: 0.1% formic acid:0.1% ammonia water:methanol (40%:30%:30%); rate of eluent: 0.5 mL/min; injection amount: 20 μL; column: X Bridge C18 5 μm, 4.6 × 250 mm; column temperature: 40°C; UV detection wavelength: 254 nm; and QDa detection: negative scan Total ion, 10V. As pyrroloquinoline quinone disodium used as a raw material of IPQ, Bio PQQ manufactured by Mitsubishi Gas Chemical Company, Inc. was used. As other compounds, reagents manufactured by Wako Pure Chemical Industries Ltd. were used unless otherwise stated.

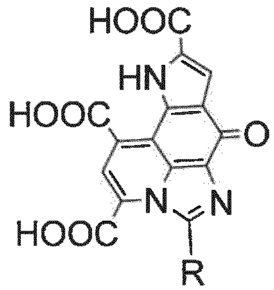

・・・Formula 1

wherein R represents an amino residue.

In the present examples, daphnids (scientific name: *Daphnia magna*) were used in a lipid reduction promotion test. As chlorella (scientific name: *Chlorella vulgaris*) used as a feed for the daphnids, a product of Nikkai Center LLC was used. Specifically, parent daphnids were bred in a water tank at 22°C under a day cycle of an 8/16-hour light/dark cycle. The chlorella used as the feed was supplied in an amount of $8 \times 10^7$ on Monday, Wednesday, and Friday. Breeding water held in the water tank was exchanged at a frequency of once a week. Under these breeding conditions, child daphnids born from the parent daphnids were used in the following examples.

Analysis of nutrient components of the chlorella used as the feed for the daphnids were requested of Shokukanken Inc., and the results were as follows:

[Table 1]

| Test Results | | | |
|---|---|---|---|
| Test Item | Test Results | Unit | Analysis Method |
| Moisture | 88.8 | g/100g | Reduced-pressure Heat Drying Method (Drying Aid Method) |
| Protein | 6.6 | g/100g | Macro Modified Kjeldahl Method |
| Lipid | 1.7 | g/100g | Acid Decomposition Method |
| Carbohydrate | 2.2 | g/100g | Calculated by Equation note 1) |
| Ash | 0.7 | g/100g | Direct Ashing Method |
| Sodium | 154 | mg/100g | ICP Emission Spectrometry |
| Salt Equivalent | 0.391 | g/100g | Calculated based on Sodium Amount |
| Energy (Calorie) | 51 | kcal/100g | Corrected Atwater Method note 2) |

[Production Example 1: IPQ Trisodium (R = glycine residue)]

100 g of PQQ disodium and 200 g of glycine were mixed with 0.5 L of water (pH 4.8). Thirty minutes after the mixing, the resultant mixture became solidified while forming bubbles. The mixture was melted by heating at 70°C, followed by a reaction for 3 days. To the thus obtained reaction liquid, 1000 g of 10 wt% NaCl water was added, followed by filtering a solid component.

The thus obtained solid component was mixed with 120 g of 25 wt% NaOH water and 30 g of water (pH 10.8), the resultant was heated to 70°C, and then allowed to stand still overnight, the resultant was cooled to recrystallize a solid component, and the solid component was filtered, and washed with 2-propanol and ethanol.

The solid component having been washed was mixed again with 500 ml of water and 41.5 g of NaOH (pH 10.3), the resultant was heated to 70°C, and then allowed to stand still overnight, the resultant was cooled to recrystallize a solid component, and the solid component was filtered, and washed with 2-propanol. The thus obtained solid component was dried under reduced pressure at 70°C, and finally, 65.8 g of glycine residue IPQ trisodium was obtained. Hereinafter, this product is also referred to as "H-IPQ". H-IPQ had a molecular weight of 341, and was identified by LC-MS analysis. As illustrated in Figure 3, [M-H] ion was detected at *m*/*z* 340.

[Production Example 2: Arginine Residue IPQ Disodium (R = arginine residue)]

2 g of PQQ disodium and 2 g of arginine were mixed with 18 g of water, followed by a reaction in air at room

temperature for 2 days. A deposition was collected from the resultant reaction liquid, and washed with ethanol, and thus, 1.92 g of arginine residue IPQ disodium in the form of a solid was obtained. Hereinafter, this product is also referred to as "Arg-IPQ". Arg-IPQ had a molecular weight of 440, and was identified by LC-MS analysis. As illustrated in Figure 4, [M-H] ion and [M-COOH] ion were respectively detected at *m*/*z* 439 and *m*/*z* 395.

[Production Example 3: Leucine Residue IPQ (R = leucine residue)]

0.5 g of PQQ disodium and 2 g of leucine were mixed with 200 g of water, followed by a reaction in air at room temperature for 3 days. To the resultant reaction liquid, 3 mL of concentrated hydrochloric acid was added to collect a deposition. The thus obtained deposition was dissolved in 50 ml of a buffer of pH 9, hydrochloric acid was added thereto to set the pH value of the buffer to 1, and thus, a solid component was recrystallized. The thus obtained solid component was washed with ethanol to obtain 0.164 g of leucine residue IPQ in the form of a solid. Hereinafter, this product is also referred to as "Leu-IPQ". Leu-IPQ had a molecular weight of 397, and was identified by LC-MS analysis. As illustrated in Figure 5, [M-H] ion and [M-COOH] ion were respectively detected at *m*/*z* 396 and *m*/*z* 352.

[Example 1] Breeding in the presence of IPQ

10 child daphnids were bred in 500 mL of mineral water (at 22°C) under a day cycle of an 8/16-hour light/dark cycle. Chlorella used as the feed was supplied in an amount of $8 \times 10^5$ to $9 \times 10^5$/day per daphnid every day. 24 hours after starting the breeding, H-IPQ was added in a concentration of 50 μM or 200 μM to the breeding water, and the chlorella used as the feed was continuously supplied in an amount of $8 \times 10^5$ to $9 \times 10^5$/day per daphnid every day. 4 days after starting the addition of H-IPQ to the breeding water, fat of the daphnids was dyed with Nile red.

[Comparative Example 1] Breeding under condition of No IPQ

10 child daphnids were bred in 500 mL of mineral water (at 22°C) under a day cycle of an 8/16-hour light/dark cycle. Chlorella used as the feed was supplied in an amount of $8 \times 10^5$ to $9 \times 10^5$/day per daphnid every day. 5 days after starting the breeding, fat of the daphnids was dyed with Nile red.

[Example 2] Breeding in the presence of Arg-IPQ

10 child daphnids were bred in 500 mL of mineral water (at 22°C) under a day cycle of an 8/16-hour light/dark cycle. Chlorella used as the feed was supplied in an amount of $8 \times 10^5$ to $9 \times 10^5$/day per daphnid every day. 24 hours after starting the breeding, Arg-IPQ was added in a concentration of 80 mg/L to the breeding water, and the chlorella used as the feed was continuously supplied in an amount of $8 \times 10^5$ to $9 \times 10^5$/day per daphnid every day. 4 days after starting the addition of Arg-IPQ to the breeding water, fat of the daphnids was dyed with Nile red.

[Example 3] Breeding in the presence of Leu-IPQ

10 child daphnids were bred in 500 mL of mineral water (at 22°C) under a day cycle of an 8/16-hour light/dark cycle. Chlorella used as the feed was supplied in an amount of $8 \times 10^5$ to $9 \times 10^5$/day per daphnid every day. 24 hours after starting the breeding, Leu-IPQ was added in a concentration of 80 mg/L to the breeding water, and the chlorella used as the feed was continuously supplied in an amount of $8 \times 10^5$ to $9 \times 10^5$/day per daphnid every day. 4 days after starting the addition of Leu-IPQ to the breeding water, fat of the daphnids was dyed with Nile red.

[Comparative Example 2] Breeding in the presence of Epigallocatechin Gallate (ECGC)

10 child daphnids were bred in 500 mL of mineral water (at 22°C) under a day cycle of an 8/16-hour light/dark cycle. Chlorella used as the feed was supplied in an amount of $8 \times 10^5$ to $9 \times 10^5$/day per daphnid every day. 24 hours after starting the breeding, ECGC was added in a concentration of 1 mg/L to the breeding water, and the chlorella used as the feed was continuously supplied in an amount of $8 \times 10^5$ to $9 \times 10^5$/day per daphnid every day. 4 days after starting the addition of ECGC to the breeding water, fat of the daphnids was dyed with Nile red.

[Comparative Example 3] Breeding in the presence of Carnitine

10 child daphnids were bred in 500 mL of mineral water (at 22°C) under a day cycle of an 8/16-hour light/dark cycle. Chlorella used as the feed was supplied in an amount of $8 \times 10^5$ to $9 \times 10^5$/day per daphnid every day. 24 hours after starting the breeding, carnitine was added in a concentration of 2 mg/L to the breeding water, and the chlorella

used as the feed was continuously supplied in an amount of 8 × $10^5$ to 9 × $10^5$/day per daphnid every day. 4 days after starting the addition of carnitine to the breeding water, fat of the daphnids was dyed with Nile red.

[Measurement of Fat Mass]

The fat mass was measured in accordance with Non Patent Literature 4. The body lengths of the daphnids bred in each of Examples 1 to 3 and Comparative Examples 1 to 3 were measured under a microscope, and the daphnids were added to 2 mL of an aqueous solution containing 0.5 mg/L of Nile red to be treated for 1 hour. Thereafter, the daphnids were washed with water, and taken out. Water was added thereto in an amount of 300 μL per daphnid, and the resultant was ground by applying ultrasonic waves for 15 minutes. The thus obtained ground liquid was centrifuged to collect a supernatant. The resultant was measured for a fluorescence intensity through 485 nm excitation/535 nm detection using a plate reader. This measurement operation was performed on 5 daphnids each. Results of comparison in body fat mass based on the fluorescence intensity are shown in Table 2. It is noted that Table 2 shows the fluorescence intensities of Examples 1 to 3 and Comparative Examples 2 to 3 with the fluorescence intensity of Comparative Example 1 used as a reference (100).

[Table 2]

| Body Fat Mass vs Comparative Example 1 (%) | | | | | | |
|---|---|---|---|---|---|---|
| | Example 1 | Example 2 | Example 3 | Comparative Example 1 | Comparative Example 2 | Comparativ e Example 3 |
| Average | 60.3 | 79.7 | 69.9 | 100.0 | 102.0 | 133.8 |
| Standard Deviation | 4.8 | 10.6 | 7.5 | 10.8 | 7.6 | 13.4 |

As shown in Table 2, the body fat mass was reduced, as compared with the control (Comparative Example 1), in Examples 1 to 3 in which H-IPQ, Arg-IPQ and Leu-IPQ were respectively added. On the other hand, in Comparative Example 3 in which carnitine was added, the body fat mass was increased, and in Comparative Example 2 in which ECGC was added, the body fat mass was neither increased not reduced.

[Measurement of Body Length]

Measurement results of the body lengths of the daphnids bred in Examples 1 to 3 and Comparative Examples 1 to 3 are shown in Table 3.

[Table 3]

| Body Length (mm) | | | | | |
|---|---|---|---|---|---|
| | Example 2 | Example 3 | Comparativ e Example 1 | Comparativ e Example 2 | Comparative Example 3 |
| Average | 2.64 | 2.52 | 2.37 | 2.43 | 2.82 |
| Standard Deviation | 0.14 | 0.18 | 0.16 | 0.02 | 0.17 |

As shown in Table 3, in Example 1 in which H-IPQ was added, the body length was not grown to be very large as compared with that of the control (Comparative Example 1), but in Examples 2 and 3 in which Arg-IPQ and Leu-IPQ were respectively added, the body length was grown to be larger, and thus, the growth promoting function was found.

[Body Fat/Body Length]

The body fat percentage of the daphnid was calculated in accordance with the following expression. It is noted that the fluorescence intensity (F. U.) of Nile red measured as described above was used instead of the actual body fat mass in the following expression. The body fat percentage of Comparative Example 1 calculated by the following expression was used as a reference (100) for evaluating the body fat percentages of Examples 1 to 3 and Comparative Examples 2 to 3. Results are illustrated in Figure 1.

$$\text{Body fat percentage (\%)} = \text{(fluorescence intensity (F. U)/body length)}$$

It is understood, as illustrated in Figure 1, that a strong fat reduction effect was exhibited in Examples 1 to 3 when the result was shown using the body fat percentage.

(Example 4) Evaluation of Movement Capability

The daphnids bred under condition of no IPQ described in Comparative Example 1 (control), and the daphnids bred in the presence of H-IPQ, Arg-IPQ, and Leu-IPQ respectively described in Examples 1 to 3 were used. 20 mL of mineral water was put in each of four 100 mm-glass petri dishes, into each of which one daphnid bred in Comparative Example 1 and Examples 1 to 3 was transferred. These petri dishes were placed to be adjacent to one another, and the movement for 1 minute of the daphnids was recorded. This operation was repeated three times using different individuals. Then, Kinovea video analysis software was used to analyze a moving distance for 30 seconds of each daphnid. Results are shown in Table 4.

[Table 4]

| | Control | IPQ | Arg-IPQ | Leu-IPQ |
|---|---|---|---|---|
| Daphnid 1 | 20.01 cm | 24.23 cm | 25.33 cm | 26.78 cm |
| Daphnid 2 | 22.67 cm | 21.43 cm | 30.35 cm | 21.56 cm |
| Daphnid 3 | 18.62 cm | 27.85 cm | 30.47 cm | 20.53 cm |
| Average ± Standard Deviation | 20.46±2.06 cm | 24.50±3.22 cm | 28.72±2.93 cm | 22.96±3.35 cm |

It is noted that the daphnids were in a state of always moving in the test, which is regarded as a state of exercise with an intensity of 3 METs or more in terms of human exercise intensity.

As described above, the amount of exercise of the daphnids was not reduced by ingesting IPQ, and on the contrary, the amount of exercise was improved by about 1.2 times by ingesting H-IPQ. When Arg-IPQ and Leu-IPQ were ingested, the amount of exercise was found to be improved respectively by 1.4 times and 1.1 times. Accordingly, there is a possibility that the motor function of the daphnids was improved by ingesting IPQ, and leucin residue or arginine residue IPQ. Besides, fat reduction can be regarded as a compound effect of influence of the increased amount of exercise and metabolic activity. It is noted that the movement of the daphnids itself was usual movement, and can be regarded as usual walking in terms of a human.

[Example 5] Correlation between Amount of Feed and Fat Mass

Daphnids were bred in the same manner as in Comparative Example 1 except that the amount of chlorella used as the feed was changed to an amount of $4 \times 10^4$/day, $2 \times 10^5$/day, or $8 \times 10^5$/day per daphnid, and fat of the daphnids was dyed with Nile red. Then, the logarithm of the amount of the feed and the fat mass (fluorescence intensity) obtained after the breeding were plotted as illustrated in Figure 2 (n = 5). When an approximate straight line was drawn on the plots, the approximate straight line was represented by the following equation, and the correlation coefficient was R = 0.996.

$$y = 227.49 \times \ln(x) - 2360.2$$

y = fat mass (fluorescence intensity) after breeding
x = amount of feed

This equation of the approximate straight line can be regarded as a relational expression between the logarithm of the amount of the feed, namely, calorie intake, and the fat mass (fluorescence intensity) after the breeding. The fat mass (fluorescence intensity) after the breeding measured in each of Examples 1 to 3 and Comparative Examples 1 to 3 was substituted for y in the above-described equation to calculate x. Assuming that the value of x in Comparative

Example 1 was x0, and that the value thereof in each experiment was xs, calculation was performed in accordance with the following equation:

$$\text{Caloric restriction equivalent amount} = 100 \times (1 - x0/x1)$$

Results are shown in Table 5. In Examples 1 to 3 and Comparative Examples 1 to 3, the amount of the actually supplied feed (calorie intake) was the same, and hence, a relative value of the value of x shown in Table 5 can be regarded as a calorie intake restriction equivalent amount against the amount of the actually supplied feed.

[Table 5]

| Caloric Restriction Equivalent Amount (0 meaning no caloric restriction) | | | | | | |
|---|---|---|---|---|---|---|
| | Exampl e 1 | Exampl e 2 | Exampl e 3 | Comparativ e Example 1 | Comparativ e Example 2 | Comparative Example 3 |
| % | 73 | 45 | 62 | 0 | -7 | -231 |

As described above, although the amount of the actually supplied feed was not reduced, and the caloric intake was not restricted, it was found that IPQs used in Examples 1 to 3 had the body fat reduction effect actually corresponding to caloric restriction of 45 to 73%.

Industrial Applicability

An agent of the present invention is industrially applicable as an agent for promoting lipid reduction or an agent for improving growth promotion.

**Claims**

1. An agent for promoting lipid reduction, comprising imidazopyrroloquinoline represented by Formula 1 and/or a salt thereof as an active ingredient:

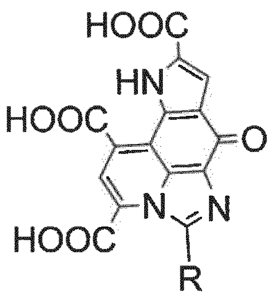

・・・Formula 1

wherein R represents an amino acid residue.

2. The agent according to claim 1, wherein R is a glycine residue, an alanine residue, an arginine residue, a histidine residue, an isoleucine residue, a leucine residue, a lysine residue, a serine residue, a threonine residue, a tryptophan residue, a tyrosine residue, or a valine residue.

3. The agent according to claim 2, wherein R is a glycine residue, a leucine residue, or an arginine residue.

4. The agent according to any one of claims 1 to 3, to be ingested during exercise with an exercise intensity of 3 METs or more, or within 1 hour before starting or within 1 hour after ending the exercise.

5. The agent according to any one of claims 1 to 3, wherein the promoting lipid reduction is body fat metabolism

promotion.

**6.** A composition, comprising the agent according to any one of claims 1 to 5.

**7.** A method for promoting lipid reduction, using the agent according to any one of claims 1 to 5.

**8.** A growth promoter, comprising imidazopyrroloquinoline represented by Formula 1, and/or a salt thereof as an active ingredient:

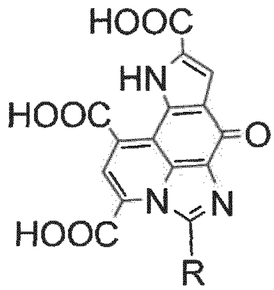

· · · Formula 1

wherein R represents an amino acid residue.

Figure 1:

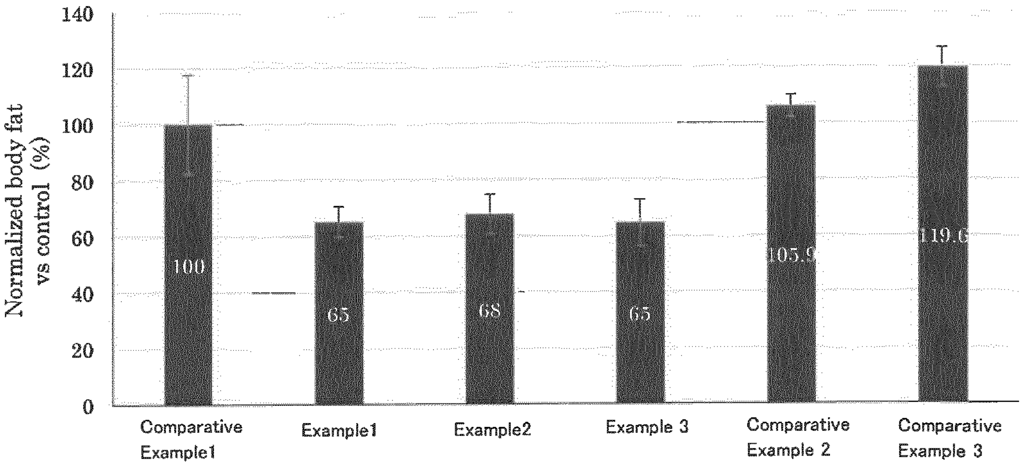


Figure 2:

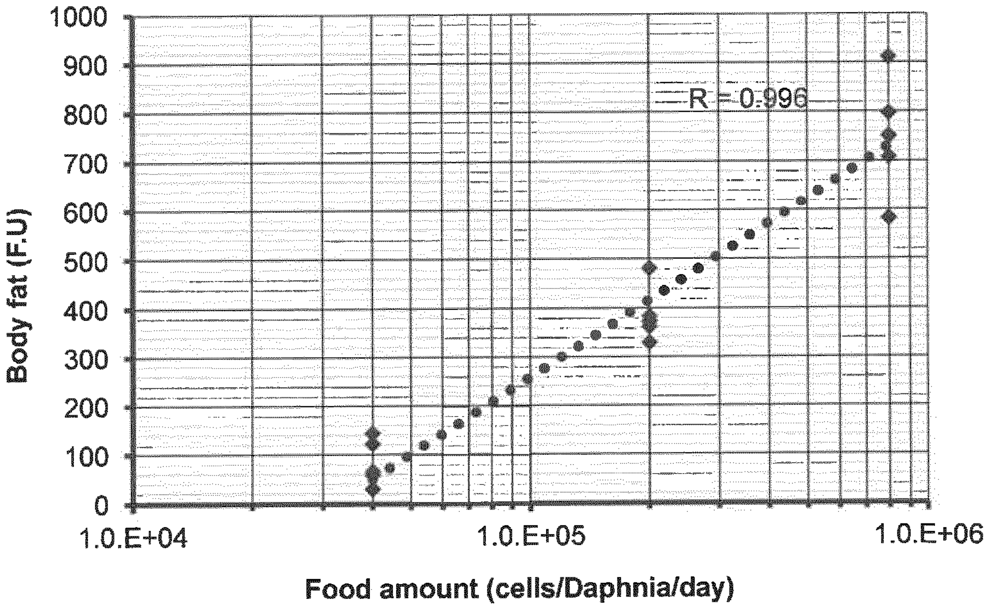


Figure 3:

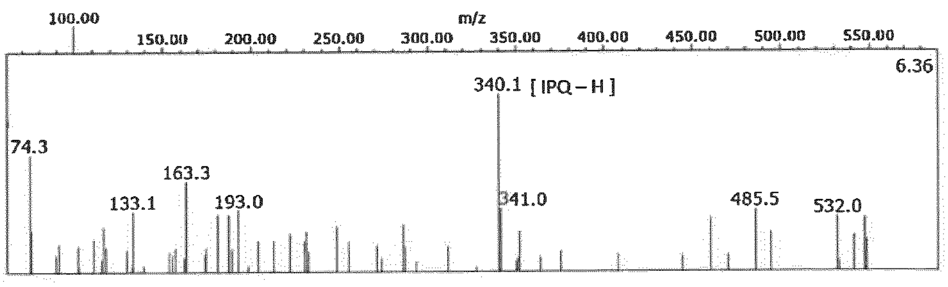

Figure 4:

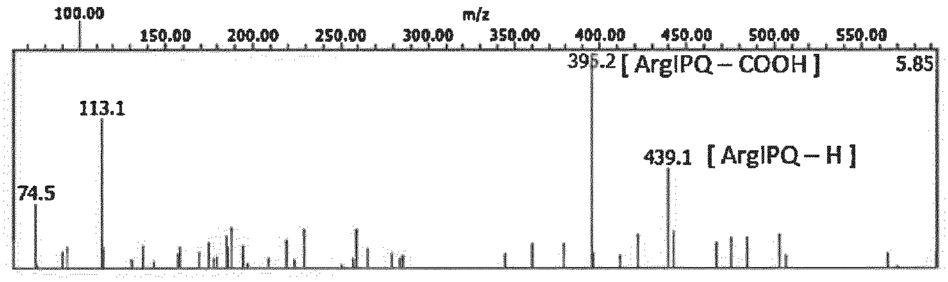


Figure 5:

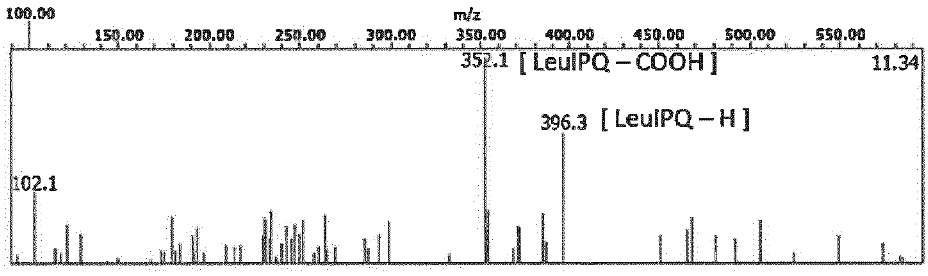

**INTERNATIONAL SEARCH REPORT**

International application No.
**PCT/JP2022/016109**

**A. CLASSIFICATION OF SUBJECT MATTER**

***A61K 31/4745***(2006.01)i; ***A61P 3/04***(2006.01)i; ***A61P 3/06***(2006.01)i; ***A61P 43/00***(2006.01)i
FI: A61K31/4745; A61P3/06; A61P3/04; A61P43/00 107

According to International Patent Classification (IPC) or to both national classification and IPC

**B. FIELDS SEARCHED**

Minimum documentation searched (classification system followed by classification symbols)

A61K31/4745; A61P3/04; A61P3/06; A61P43/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 10-45594 A (MITSUBISHI GAS CHEMICAL CO INC.) 17 February 1998 (1998-02-17) claims, paragraphs [0010], [0011], [0014], each table | 6 |
| Y | | 1-5, 7, 8 |
| X | JP 9-40675 A (MITSUBISHI GAS CHEMICAL CO INC.) 10 February 1997 (1997-02-10) claims, paragraphs [0008], [0019], [0036], example 5, paragraph [0080] | 6 |
| Y | | 1-5, 7, 8 |
| X | JP 2018-104312 A (MITSUBISHI GAS CHEMICAL CO INC.) 05 July 2018 (2018-07-05) claims, examples | 6 |
| Y | | 1-5, 7, 8 |
| X | YAMADA, Yasue et al. Effects of pyrroloquinoline quinone and imidazole pyrroloquinoline on biological activities and neural functions. Heliyon. 2020, vol. 6, e03240, https://doi.org/10.1016/j.heliyon.2020.e03240 titles, abstracts, drawings, tables, etc. | 6 |
| X | abstract, 3.2., fig. 2, etc. | 8 |
| Y | abstract, 3.2., 3.5., fig. 2, 4, etc. | 1-5, 7, 8 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 May 2022** | **07 June 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/016109**

**C. DOCUMENTS CONSIDERED TO BE RELEVANT**

| Category* | Citation of document, with indication, where appropriate, of the relevant passages | Relevant to claim No. |
|---|---|---|
| Y | MISHIMA, Tomoyuki. Metabolic fate of PQQ and effects of PQQ on lipid metabolism. 科学研究費助成事業研究成果亮告書. 15K21475, 2020, Internet: <URL: https://kaken.nii.ac.jp/grant/KAKENHI-PROJECT-15K21475/>, (Grant-in-Aid for Scientific Research, Final Research Report.)<br>1. lines 7-12, 4.(1), tables 1, 4.(2), fig. 2, etc. | 1-5, 7 |
| Y | 江崎治ほか. 食事・運動療法のサイエンス. 第124回日本医学会シンポジウム記録集. 2003, pp. 137-145, (EZAKI, Osamu, MIURA, Shinji. Molecular and biological aspects of exercise training and energy restriction), non-official translation (Proceedings of the 124th Symposium of The Japanese Association of Medical Sciences.)<br>lines 5-8 from the top, drawings | 4 |
| Y | FUJIMOTO, Eri et al. Change in PGC-1alpha Expression in Rat Skeletal Muscle after Low-intensity Prolonged Swimming Exercise. J. Physiol. Anthropol. 2011, vol. 30, no. 1, pp. 23-27<br>titles, abstracts, drawings, tables, etc. | 4 |
| A | 運動強度とエネルギー消費量. 健康長寿ネット. [online] 2019, [retrieved on 23 May 2022], Internet: <URL: https://www.tyojyu.or.jp/net/kenkou-tyoju/undou-kiso/undou-energy.html>, non-official translation (Exercise Intensity and Energy Consumption Amounts. Health and Longevity Net.)<br>"Assessment of Exercise Intensity (MET)", tables 1, 2, "Energy Consumption Amount", drawings, etc. | 4 |

Form PCT/ISA/210 (second sheet) (January 2015)

**INTERNATIONAL SEARCH REPORT**
**Information on patent family members**

International application No.
**PCT/JP2022/016109**

| Patent document cited in search report | Publication date (day/month/year) | Patent family member(s) | Publication date (day/month/year) |
|---|---|---|---|
| JP 10-45594 A | 17 February 1998 | (Family: none) | |
| JP 9-40675 A | 10 February 1997 | (Family: none) | |
| JP 2018-104312 A | 05 July 2018 | US 2018/0186794 A1<br>claims, examples | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *BioFactors,* 1995, vol. 5 (2), 75-81
- *J Cell Sci.,* 01 August 2017, vol. 130 (15), 2631-2643
- *Heliyon,* 2020, vol. 6, e03240
- *Environ Health Perspect,* 2015, vol. 123 (8), 813-819
- The Japanese Pharmacopoeia